# EUROPEAN PATENT APPLICATION

(11) **EP 3 120 877 A1**
(43) Date of publication of application: **25.01.2017**
(21) Application number: 15178300.8
(22) Date of filing: 24.07.2015
(51) Int. Cl.: A61L 31/02, A61L 31/10

(54) **ENDOLUMINAL DEVICE**

(71) Applicant: B. Braun Melsungen AG, 34212 Melsungen (DE)
(72) Inventor: CALISSE, Jorge, 12203 Berlin (DE)
(74) Representative: Patentanwälte Ruff, Wilhelm, Beier, Dauster & Partner mbB

(57) **Abstract**

The invention relates to an endoluminal device comprising a device body comprising a
- a first structure comprising at least one metal selected from the group comprising magnesium, zinc, iron and alloys thereof and
- a second second structure comprising a least one polymer selected from the group comprising polylactide, polycaprolactone, poly(trimethylene carbonate), copolymers thereof, and blends thereof.

Further, the invention relates to a medical kit or system, in particular for use in angioplasty, comprising the endoluminal device and a delivery device.

## Description

The invention relates to a device, in particular endoluminal device, preferably useful in the treatment of stenosis and preventing restenosis disorders, and to a medical kit.

Tubular organs and structures such as blood vessels are prone to stenosis, i.e. narrowing or constriction of the lumen. A stenosis can be caused by a variety auf traumatic or organic disorders and symptoms can range from mild irritation and discomfort to paralysis and death. Treatment is site specific and dependent on the nature and extent of the stenosis.

For treating a stenosis in a blood vessel or heart valve, stents are typically applied. Stents reinforce body vessels and prevent restenosis following angioplasty in the vascular system.

Stents are typically composed of scaffolding that includes a pattern of a plurality of interconnecting structural elements or struts. The stent scaffolding physically holds open and, if desired, expands the wall of the passage way. Typically, stents are capable of being compressed or crimped onto a catheter so that they can be delivered to and deployed at a treatment site.

Delivery includes inserting the stent through small lumens using a catheter and transporting it to the treatment site. Deployment includes expanding the stent to a larger diameter once it is at the desired location.

As an example, an endovascular stent may be inserted into a blood vessel during angioplasty, and is designed to prevent early collapse of a vessel that has been weakened or damaged by angioplasty. Insertion of endovascular stents has been shown to reduce negative remodeling of the vessel while healing of the damaged vessel wall proceeds over a period of months.

During the healing process, inflammation caused by angioplasty and stent device injury often causes smooth muscle cell proliferation and regrowth inside the stent, thus partially closing the flow channel, and thereby reducing or eliminating the beneficial effect of the angioplasty/stenting procedure. This process is called restenosis. Blood clots may also form inside of the newly implanted stent due to the thrombotic nature of the stent surfaces, even when biocompatible materials are used to form the stent. While large blood clots may not form during the angioplasty procedure itself or immediately post-procedure, due to the current practice of injecting powerful anti- platelet drugs into the blood circulation, some thrombosis is always present, at least on a microscopic level on stent surfaces, and it is supposed to play a significant role in the early stages of restenosis by establishing a biocompatible matrix on the surfaces of the stent where upon smooth muscle cells may subsequently migrate in and proliferate.

Stents can be of a permanent or temporary nature. Temporary stents may be advantageous, particular in cases of recurrent vessel narrowing in which it is desirable to insert a subsequent stent at or near the site of initial stent placement, or where a stent is needed only temporarily to counteract post-surgical swelling that may cause obstruction of a bodily lumen.

Stents made of a biodegradable material such as polylactide are known from EP 2 647 394 A2, EP 2 647 484 A1, WO 2000/074744 A1, WO 2007/139668 A2, EP 2 559 408 A2, US 2013/0317590 A1, WO 2007/126598 A2 and EP 2 465 551 A2.

Biodegradable polymeric stents often exhibit good stiffness which results in a long-term patency rate. On the other hand, these stents principally suffer from an increased recoil risk. Recoil refers to the percentage by which the diameter of a stent decreases from its expanded diameter (e.g. when a balloon is inflated at a nominal pressure) to its relaxed diameter (e.g. when a balloon is retrieved from the stent).

Further, biodegradable polymeric stents may expose an insufficient adhesion to a delivery catheter which may impede a medically correct placement of the stents. In case of stents made of polylactide, there is typically only a time slot of around 3 minutes for the surgeon to deliver and place the stents in a bodily lumen portion to be treated.

Stents formed from a magnesium alloy are known from WO 2013/052856 A2, WO 2013/024124 A1, WO 2011/117298 A1 and EP 1 632 255 A2. These stents exhibit superior mechanical properties in comparison to biodegradable polymeric stents. However, stents of magnesium alloy have their limitations with respect to a long-term patency rate. In that regard, mere administration of anti-proliferative agents is not suited to compensate for that shortcoming.

Thus, there remains a clinical need for devices which circumvent the disadvantages associated with common stents. In particular, there is a specific need of devices which on the one hand minimize the risk of recoil but on the other hand ensure a long-term patency rate.

### OBJECT AND SOLUTION

Thus, the object underlying the present invention is to provide a device which properly addresses the afore-mentioned need.

This object is solved by a device having the features of independent claim 1. Preferred embodiments of the device are defined in the dependent claims 2 to 15. Further, the object is solved by a medical kit or system as described in the description. The wording of all of the claims is incorporated into the description by reference.

According to a first aspect, the invention relates to a device, preferably an endoprosthetic or endoluminal device, comprising a device body, i.e. a substrate, backbone or scaffolding.

The device body comprises a first structure and a second structure.

The first structure comprises at least one metal, preferably at least one bioerodible metal, which is preferably selected from the group comprising magnesium, zinc, iron and alloys thereof.

The second structure comprises at least one polymer, preferably at least one bioerodible polymer, which is preferably selected from the group comprising polylactide, polycaprolactone, poly(trimethylene carbonate), polyhydroxyalkanoates, polyglycolide, polyhydroxybutyrate poly(3-hydroxybutyrate), poly(4-hydroxybutyrate), copolymers thereof, and blends thereof.

The term "endoprosthetic device" or "endoluminal device" as used according to the present invention refers to an artificial device that is adapted or configured to be implanted in a bodily lumen, in particular vascular lumen, preferably an arterial lumen. Thus, the device, in particular endoprosthetic or endoluminal device, according to the present invention may also be denoted as an implant, in particular endoprosthetic or endoluminal implant.

The term "lumen" as used according to the present invention refers to a cavity of an organ, in particular of a tubular organ such as a blood vessel, preferably artery.

The term "bodily lumen" as used according to the present invention means a lumen within a human or animal body. Preferably, the term "bodily lumen" as used according to the present invention means a vascular lumen, in particular an arterial or venous lumen, more preferably an arterial lumen.

The term "bioerodible" or "biodegradable" means according to the present invention disintegration upon exposure to bodily fluids, preferably blood, and can include gradually resorption, absorption and/or elimination by a patient's body.

The term "copolymer" as used according to the present invention defines a polymer which comprises, in particular consists of, at least two different monomeric units. Thus, a copolymer according to the present invention can be, by way of example, a bipolymer or terpolymer. For example, the copolymers mentioned in the context of the second structure can comprise, in particular consist of, monomeric units, which are selected from the group comprising hydroxyalkanoate units, trimethylene carbonate units and combinations thereof, and at least one further type of monomeric units. The at least one further type of monomeric units can also be selected from the group comprising hydroxyalkanoate units, trimethylene carbonate units and combinations thereof or can be a different type of monomeric units. The hydroxyalkanoate units are preferably selected from the group comprising lactic acid units, glycolic acid units, hydroxybutyrate units, 3-hydroxybutyrate units, 4-hydroxybutyrate units, caprolactone units (caproic acid units) and combinations thereof.

The term "monomeric units" as used according to the present invention encompasses not only a plurality of monomeric units, i.e. two or more monomeric units, but can also mean one monomeric unit included in the copolymers. Accordingly, the term "hydroxyalkanoate units" as used according to the present invention encompasses not only a plurality of hydroxyalkanoate units, i.e. two or more hydroxyalkanoate units, but can also mean only one hydroxyalkanoate unit included in the copolymers, while the term "trimethylene carbonate units" as used according to the present invention encompasses not only a plurality of trimethylene carbonate units, i.e. two or more trimethylene carbonate units, but can also mean only one trimethylene carbonate unit included in the copolymers. The same applies correspondingly to the examples given for the hydroxyalkanoate units.

The term "blend" as used according to the present invention defines a polymer mixture which comprises, in particular consists of, at least two different polymers. For example, the blends disclosed in the context of the second structure can comprise, in particular consist of, a polymer, which is selected from the group comprising polylactide, polycaprolactone, poly(trimethylene carbonate), polyhydroxyalkanoates, polyglycolide, polyhydroxybutyrate, poly(3-hydroxybutyrate), poly(4-hydroxybutyrate), copolymers thereof and combinations thereof, and at least one further type of polymer. The at least one further type of polymer can also be selected from the group mentioned in this paragraph or can be, alternatively, a different type of polymer.

The term "alloy" as used according to the present invention defines a mixture of at least two different metals. For example, the alloys disclosed in the context of the first structure, can comprise, in particular consist of, a metal, preferably bioerodible metal, which is selected from the group comprising magnesium, zinc and iron, and at least one further metal. The at least one further metal can also be selected from the metal group mentioned in this paragraph or can be, alternatively, a different metal. With respect to different metals, reference is made to the at least one further metal disclosed in the context of magnesium alloys in the present description.

The invention is particularly featured by the following advantages:
- The device according to the present invention combines the advantages of conventional metallic stents, in particular magnesium or magnesium alloy stents, and biodegradable polymeric stents, in particular stents of polylactide.
- The first structure advantageously exhibits mechanical properties of a metallic stent such as radial strength and fracture toughness. Thus, a recoil or collapse risk can be minimized, in particular during an initial stage, e.g. after removal of delivery device, preferably after deflation of a balloon catheter.
- Further, retention on a delivery device, in particular on a balloon of a balloon catheter, before and during implantation can be improved. Thus, delivery of the device to a target lesion is made more convenient, resulting in a better handling for the surgeon and a higher safety for the patient.
- Due to the stiffness and strength of the at least one polymer, in particular polylactide, the second structure advantageously facilitates a long-term patency rate which is highly desirable under medical point of views.
- Further advantages may arise from using polycaprolactone and/or poly(trimethylene carbonate), especially if transferred into a memory shape condition, which may also contribute to a long-term patency rate.

The device according to the present invention may consist of the device body, i.e. the substrate, backbone or scaffolding.

In a preferred embodiment, the device, in particular device body, is bioerodible or biodegradable.

In a further embodiment, the first structure is bioerodible or biodegradable. In a further embodiment, the second structure is bioerodible or biodegradable.

In a further embodiment, the first structure has a shorter bioerosion or biodegradation time than the second structure. In other words, the second structure preferably has a longer bioerosion or biodegradation time than the first structure. The shorter bioerosion or biodegradation time of the first structure is preferably due or basically due to the at least one metal. The longer bioerosion or biodegradation time of the second structure is preferably due or basically due to the at least one polymer. Thus, the first structure preferably increases the device's mechanical stability during an initial and medium-term stage, in particular during storage (e.g. when crimped onto a delivery catheter), during implantation and in particular during a couple of months after implantation, while the second structure advantageously facilitates a long-term support of the treated bodily lumen portion, and thus is advantageously responsible for a long-term patency rate. Further, due to a preferably permanent erosion or degradation of the first structure, a continuously increasing organ apposition, in particular blood vessel apposition, preferably artery apposition, can be accomplished, due to an increasing expansion of the second structure into the wall containing the bodily lumen.

The first structure may undergo bioerosion or biodegradation within a couple of months. For example, the first structure may have a bioerosion rate or biodegradation rate of 1 to 4 months.

The second structure preferably undergoes bioerosion or biodegradation within a couple years. For example, the second structure may have a bioerosion rate or biodegradation rate of 1 to 3 years.

In a preferred embodiment, the first structure is arranged at a luminal side of the device, in particular device body. Thus, an erosion or degradation of the first structure can be accelerated. A fast erosion or degradation of the first structure may further be facilitated by a bodily liquid, preferably blood, flowing through the lumen which immediately removes erosion or degradation products of the first structure.

The term "luminal side of the device, in particular device body" as used according to the present invention means a side of the device, in particular device body, which - after implantation of the device, in particular device body, - faces a bodily lumen or is directed to a bodily lumen. Thus, the luminal side of the device, in particular device body, may also be denoted as the interior side of the device, in particular device body.

In a further embodiment, the first structure is arranged at a luminal side of the second structure.

The term "luminal side of the second structure" as used according to the present invention means the side of the second structure which - after implantation of the device, in particular device body, - is directed to a bodily lumen.

In a further embodiment, the first structure and the second structure may have a same physical form.

In an alternative embodiment, the first structure and the second structure may have a different physical form.

The physical form of the first structure and second structure may be independently chosen from a non-textile form and/or a textile form.

Thus, it may be conceivable within the scope of the present invention, that the first structure and the second structure each have a non-textile form. In that regard, the first structure and the second structure may have the same non-textile form or a different non-textile form.

Alternatively, the first structure and the second structure each may have a textile form. In that regard, the first structure and the second structure may have the same textile form or a different textile form.

Alternatively, the first structure may have a non-textile form and the second structure may have a textile form.

Alternatively, the first structure may have a textile form and the second structure may have a non-textile form.

With respect to suitable non-textile forms and textile forms, reference is made to the following description.

In a further embodiment, the first structure comprises or is a non-textile structure. The nontextile structure is in particular selected from the group comprising a film, a layer, a sheet, a coating, a membrane and a lattice.

In a further embodiment, the first structure comprises or is a layer or sheet.

According to an alternative preferred embodiment, the first structure comprises or is a lattice structure.

In a further embodiment, the first structure comprises or is at least one filamentary element.

Preferably, the first structure comprises or is a textile structure, in particular comprising at least one filamentary element.

The term "at least one filamentary element" as used according to the present invention means one filamentary element, in particular only one filamentary element, or a plurality of filamentary elements, i.e. two or more filamentary elements.

The at least one filamentary element is in particular selected from the group comprising monofilament, staple fiber, pseudo monofilament, multifilament (such as twisted or braided multifilament), thread, yarn, wire, and combinations thereof.

The at least one filamentary element may have a diameter from 5 µm to 100 µm, in particular 20 µm to 50 µm.

Further, the at least one filamentary element may have a circular cross-section.

Alternatively, the at least one filamentary element may have a non-circular cross-section such as a trigonal cross-section, square cross-section, trapezoidal cross-section, rhombic cross-section, pentagonal cross-section, hexagonal cross-section or star-like cross-section.

A circular or triangular cross-section is preferable, since both types of cross-sections allow for a better embedding of the device, in particular device body, in a wall surrounding a bodily lumen, and thus facilitate a secure and in particular long-term positioning of the device within a bodily lumen.

In a further embodiment, the first structure comprises a plurality of filamentary elements, wherein the filamentary elements are aligned parallel to each other.

In a further embodiment, the first structure comprises a plurality of filamentary elements, wherein the filamentary elements are twisted or braided.

In a further embodiment, the first structure comprises a plurality of filamentary elements, wherein a part of the filamentary elements has a counterclockwise turning, while the remaining part of the filamentary elements has a clockwise turning.

In a further embodiment, the first structure comprises 1 to 1000 filamentary elements, preferably 10 filamentary elements.

In a further embodiment, the first structure comprises or is a textile structure. The textile structure is in particular selected from the group comprising woven textile or woven fabric, knit or knitted fabric, non-woven fabric, braid (braiding), mesh, textile lattice or textile grid and combinations thereof.

Preferably, the first structure comprises or is a knit or knitted fabric.

The knitted fabric may be in particular selected from the group comprising warp-knitted fabric, locknit fabric (also referred to as tricot or jersey knitted fabric), reverse locknit fabric, shark skin knitted fabric, queenscord knitted fabric and velour knitted fabric.

According to an alternative preferred embodiment, the first structure comprises or is a braid.

According to an alternative preferred embodiment, the first structure comprises or is a textile lattice or textile grid.

In a further embodiment, the first structure may have a cylindrical, in particular tubular, shape.

In an alternative embodiment, the first structure may have a spiral or helical, in particular helical, shape.

In a further embodiment, the first structure comprises magnesium and/or a magnesium alloy.

The term "magnesium alloy" as used according to the present invention defines a mixture of magnesium (typically as the main constituent) with at least one further metal. The at least one further metal may be selected from the group comprising aluminum, bismuth, copper, cadmium, rare earths such as gadolinium and/or yttrium, iron, thorium, strontium, zirconium, lithium, manganese, nickel, lead, silver, chromium, silicon, tin, calcium, antimony, zinc, and combinations thereof.

For example, the metal alloy may be an alloy which is commercially available under the abbreviation "AZ31". This magnesium alloy contains 3.3 to 4.0 percent by weight of aluminum, 0.25 to 0.50 percent by weight of manganese, 0.05 to 0.20 percent by weight of zinc, at most 0.003 percent by weight of iron, at most 0.02 percent by weight of copper, at most 0.10 percent by weight of silicon and at most 0.002 percent by weight of nickel, each in relation to the total weight of the magnesium alloy.

A further useful magnesium alloy is commercially available under the abbreviation "AZ61". This magnesium alloy contains 5.92 percent by weight of aluminum, 0.49 percent by weight of zinc, 0.15 percent by weight of manganese, 0.037 percent by weight of silicon, 0.003 percent by weight of copper and 0.007 percent by weight of iron, each in relation to the total weight of the magnesium alloy.

A further useful magnesium alloy is commercially available under the abbreviation "AZ91". This magnesium alloy contains 9 percent by weight of aluminum, 1.0 percent by weight of zinc and 0.3 percent by weight of manganese, each in relation to the total weight of the magnesium alloy.

A further useful magnesium alloy is commercially available under the abbreviation "WE43". This magnesium alloy contains 3.7 to 4.3 percent by weight of yttrium, 2.4 to 4.4 percent by weight of rare earths and 0.4 percent by weight of zirconium, each in relation to the total weight of the magnesium alloy.

In a further embodiment, the first structure may consist of at least one metal, preferably at least one bioerodible metal, which is selected from the group comprising magnesium, zinc, iron and alloys thereof, and optionally of additives. With respect to the at least one metal, reference is made to the previous description. With respect to useful additives, reference is made to the following description.

According to an especially preferred embodiment, the second structure comprises polylactide and/or a copolymer containing lactide units.

The polylactide is preferably selected from the group comprising poly(L-lactide), poly(D,L-lactide), poly(D-lactide) and mixtures thereof.

More preferably, the polylactide is poly(L-lactide).

In a further embodiment, the copolymers mentioned in the context of the second structure are selected from the group comprising poly(L-lactide-co-glycolide), poly(D,L-lactide-co-glycolide), poly(D-lactide-co-glycolide), poly(L-lactide-co-caprolactone), poly(D,L-lactide-co-caprolactone), poly(D-lactide-co-caprolactone), poly(3-hydroxybutyrate-co-3-hydroxyvalerate), poly(3-hydroxybutyrate-co-4-hydroxybutyrate), poly(3-hydroxybutyrate-co-3-hydroxyoctanoate) and mixtures thereof.

In a further embodiment, the second structure may consist of at least one polymer, which is selected from the group comprising polylactide, polycaprolactone, poly(trimethylene carbonate), polyhydroxyalkanoates, polyglycolic acid, polyhydroxybutyrate, poly(3-hydroxybutyrate), poly(4-hydroxybutyrate), copolymers thereof, and blends thereof, and optionally of additives. With respect to the at least one polymer, reference is made to the previous description. With respect to useful additives, reference is made to the following description.

Preferably, the at least one polymer disclosed in the context of the second structure has a low crystallinity. Specifically, the at least one polymer may have crystallinity from 0.01 percent by weight to 40 percent by weight, in particular 0.02 percent by weight to 20 percent by weight, preferably 0.05 percent by weight to 10 percent by weight. Low crystallinity, in particular the crystallinity values disclosed in this paragraph, have advantages in respect of the degradation process and in respect of biocompatibility during the polymer bioerosion process in a human or animal body.

In an alternative embodiment, the at least one polymer disclosed in the context of the second structure is complete amorphous.

In a further embodiment, the second structure comprises or is a non-textile structure. The non-textile structure is in particular selected from the group comprising a film, a layer, a sheet, a coating, a membrane and a lattice.

Preferably the second structure comprises or is a layer or sheet.

According to an alternative preferred embodiment, the second structure comprises or is a lattice structure.

Preferably, the second structure comprises or is at least one filamentary element.

In an alternative embodiment, the second structure comprises or is a textile structure, in particular comprising at least one filamentary element.

The at least one filamentary element is in particular selected from the group comprising mono-filament, staple fiber, pseudo monofilament, multifilament (such as twisted or braided multifila-ment), thread, yarn, wire and combinations thereof.

The at least one filamentary element may have a diameter from 5 µm to 100 µm, in particular 20 µm to 50 µm.

Further, the at least one filamentary element may have a circular cross-section.

Alternatively, the at least one filamentary element may have a non-circular cross-section such as a trigonal cross-section, square cross-section, trapezoidal cross-section, rhombic cross-section, pentagonal cross-section, hexagonal cross-section or star-like cross-section.

A circular or triangular cross-section is preferable, since both types of cross-sections allow for a better embedding of the device, in particular device body, in a wall surrounding a bodily lumen, and thus facilitate a secure and in particular long-term positioning of the device within a bodily lumen.

In a further embodiment, the second structure comprises a plurality of filamentary elements, wherein the filamentary elements are aligned parallel to each other.

In a further embodiment, the second structure comprises a plurality of filamentary elements, wherein the filamentary elements are twisted or braided.

In a further embodiment, the second structure comprises a plurality of filamentary elements, wherein a part of the filamentary elements has a clockwise turning, while the remaining part of the filamentary elements has a counterclockwise turning.

In a further embodiment, the second structure comprises 1 to 1000 filamentary elements, preferably 10 filamentary elements.

In a further embodiment, the second structure comprises or is a textile structure. The textile structure is in particular selected from the group comprising woven textile or woven fabric, knit or knitted fabric, non-woven fabric, braid (braiding), mesh and combinations thereof.

Preferably, the second structure comprises or is a knit or knitted fabric.

The knitted fabric may be in particular selected from the group comprising warp-knitted fabric, locknit fabric (also referred to as tricot or jersey knitted fabric), reverse locknit fabric, shark skin knitted fabric, queenscord knitted fabric and velour knitted fabric.

According to an alternative preferred embodiment, the second structure comprises or is a braid.

According to an alternative preferred embodiment, the second structure comprises or is a textile lattice or textile grid.

Principally, the first structure may be coated, wrapped, enwrapped, plaited round (braided) or enwound by the second structure.

However, preferably, the second structure is coated, wrapped, enwrapped, plaited round (braided) or enwound by the first structure. Thus, it can be facilitated that the first structure erodes or degrades at first, while erosion or degradation of the second structure takes not place until degradation of the first structure has at least been initiated.

In a further embodiment, the first structure and the second structure are arranged in a common textile weave. The weave may be selected from the group comprising a knitting weave, a braiding weave (intertwining or interlacing weave), a plane weave, a warp-twill weave, weaved twill weave, broad rib-twill weave, multiple rib-twill weave, satin weave, warp satin weave, weaved satin weave and combinations thereof.

In a further embodiment, the first structure and the second structure are joined together by means of material-locking, in particular by gluing, welding, and the like.

Preferably, the first structure and the second structure are joined together by means of a connecting material and/or an anti-proliferative coating or layer as described in the following.

In an alternative embodiment, the first structure and the second structure are joined together without the aid of a connecting material, in particular glue, as described in the following.

In a further embodiment, the second structure is arranged at an abluminal side of the device, in particular device body.

The term "abluminal side of the device, in particular device body" as used according to the present invention means a side of the device, in particular device body, which - after implantation of the device, in particular device body, - is away from a bodily lumen, i.e. is directed towards a wall containing a bodily lumen. Thus, the abuminal side may also be denoted as the exterior side of the device, in particular device body.

In a further embodiment, the second structure is arranged on an abluminal side of the first structure.

The term "abluminal side of the first structure" as used according to the present invention means the side of the first structure which - after implantation of the device, in particular device body, - is away from a bodily lumen, i.e. is directed to a wall containing a bodily lumen.

In a further embodiment, the first structure and the second structure are twisted together, in particular in the manner of a steel cable construction. In that case, the first structure and the second structure may be alternately arranged at a luminal side of the device, in particular device body, and an abluminal side of the device, in particular device body.

In a further embodiment, the first structure and the second structure are braided together.

In a further embodiment, the second structure may have a cylindrical, in particular tubular, shape.

In an alternative embodiment, the second structure may have a spiral or helical, in particular helical, shape.

In a further embodiment, the device body may consist of the first structure, the second structure and optionally of additives. With respect to the first and second structure, reference is made to the previous description. With respect to useful additives, reference is made to the following description.

In a further embodiment, the device further comprises a third structure comprising a connecting material.

The term "connecting material" as used according to the present invention means a material that is able to connect, i.e. to join, the first and the second structure with each other. Preferably, the connecting material is an adhesion promoting material, or a glue.

It may be in particular envisaged according to the present invention that the third structure consists of the connecting material.

The third structure advantageously prevents that particles of the first structure, in particular magnesium or magnesium alloy particles, are released in the interior of a bodily lumen before bioerosion or biodegradation of the first structure has begun.

Preferably, the third structure is bioerodable or biodegradable.

It may be in particular envisaged that the third structure has a lower bioerosion or biodegradation rate than the first structure, but preferably a faster bioerosion or biodegradation rate than the second structure.

Preferably, the third structure is arranged at an abluminal side of the first structure.

More preferably, the third structure is arranged between the first structure and the second structure.

It is especially preferred that the first structure and the second structure are joined together by the third structure.

Preferably, the third structure is arranged at an abluminal side of the first structure and is concurrently arranged at a luminal side of the second structure.

In a further embodiment, the third structure comprises or is a non-textile structure. The non-textile structure is in particular selected from the group comprising a film, a layer, a sheet, a coating, a membrane, a lattice and combinations thereof.

Preferably, the third structure comprises or is a coating, layer or sheet.

According to an alternative preferred embodiment, the third structure comprises or is a lattice structure.

In a further embodiment, the third structure comprises or is at least one filamentary element.

In a further embodiment, the third structure comprises or is a textile structure, in particular comprising at least one filamentary element.

The at least one filamentary element may be in particular selected from the group comprising monofilament, staple fiber, pseudo monofilament, multifilament (such as twisted or braided multifilament), thread, yarn, wire and combinations thereof.

The at least one filamentary element may have a diameter from 5 µm to 100 µm, in particular 20 µm to 50 µm.

Further, the at least one filamentary element may have a circular cross-section.

Alternatively, the at least one filamentary element may have a non-circular cross-section such as a trigonal cross-section, square cross-section, trapezoidal cross-section, rhombic cross-section, pentagonal cross-section, hexagonal cross-section or star-like cross-section.

A circular or triangular cross-section is preferable, since both types of cross-sections allow for a better embedding of the device, in particular device body, in a wall surrounding a bodily lumen, and thus facilitate a secure and in particular long-term positioning of the device within a bodily lumen.

In a further embodiment, the third structure comprises a plurality of filamentary elements, wherein the filamentary elements are aligned parallel to each other.

In a further embodiment, the third structure comprises a plurality of filamentary elements, wherein the filamentary elements are twisted or braided.

In a further embodiment, the third structure comprises a plurality of filamentary elements, wherein a part of the filamentary elements has a counterclockwise turning, while the remaining part of the filamentary elements has a clockwise turning.

In a further embodiment, the third structure comprises 1 to 1000 filamentary elements, preferably 10 filamentary elements.

In a further embodiment, the third structure comprises or is a textile structure. The textile structure is in particular selected from the group comprising woven textile or woven fabric, knit or knitted fabric, non-woven fabric, braid (braiding), mesh and combinations thereof.

Preferably, the third structure comprises or is a knit or knitted fabric.

The knitted fabric may be in particular selected from the group comprising warp-knitted fabric, locknit fabric (also referred to as tricot or jersey knitted fabric), reverse locknit fabric, shark skin knitted fabric, queenscord knitted fabric and velour knitted fabric.

According to an alternative preferred embodiment, the third structure comprises or is a braid.

According to a further alternative preferred embodiment, the third structure comprises or is a textile lattice or textile grid.

In a further embodiment, the first structure, second structure and third structure are arranged in a common textile weave. The weave may be selected from the group comprising a knitting weave, a braiding weave (intertwining or interlacing weave), a plane weave, a warp-twill weave, weaved twill weave, broad rib-twill weave, multiple rib-twill weave, satin weave, warp satin weave, weaved satin weave and combinations thereof.

In a further embodiment, the first structure, second structure and third structure are joined together by means of material-locking, in particular gluing, welding, and the like.

The connecting material is in particular selected from the group comprising polyhydroxyalkanoates, poly(L-lactide), poly(D,L-lactide, poly(D-lactide), poly(L-lactide-co-glycolide), poly(D,L-lactide-co-glycolide), poly(D-lactide-co-glycolide), poly(L-lactide-co-caprolactone), poly(D-lactide-co-caprolactone), poly(D,L-lactide-co-caprolactone), polyglycolide, polyhydroxybutyrate, poly(3-hydroxybutyrate), poly(4-hydroxybutyrate), poly(3-hydroxybutyrate-co-3-hydroxyvalerate), poly(3-hydroxybutyrate-co-4-hydroxybutyrate), poly(3-hydroxybutyrate-co-3-hydroxyoctanoate) and mixtures thereof.

It may be further conceivable that the connecting material is different to polylactide, preferably different to poly(L-lactide). With respect to suitable materials, reference is made to the preceding paragraph.

In a further embodiment, the third structure may have a cylindrical, in particular tubular, shape.

In an alternative embodiment, the third structure may have a spiral or helical, in particular helical, shape.

In a further embodiment, the first structure, second structure and third structure may have a same physical form.

In an alternative embodiment, the first structure, second structure and third structure each may have a different physical form.

The physical form of the first structure, second structure and third structure may be independently chosen from a non-textile form and/or a textile form. With respect to useful non-textile forms and/or textile forms, reference is made to the previous description.

In a further embodiment, the first structure, the second structure and the third structure are twisted together.

In a further embodiment, the first structure, the second structure and the third structure are braided together.

In a further embodiment, the device body may consist of the first structure, the second structure, the optional third structure and optionally of additives. With respect to the first structure, the second structure and the optional third structure, reference is made to the previous description. With respect to useful additives, reference is made to the following description.

In a further embodiment, the device, in particular device body, preferably the first structure, second structure and/or the optional third structure, further comprises an anti-proliferative agent.

The anti-proliferative agent advantageously minimizes the risk that neointimal hyperplasia, and thus restenosis occurs.

More preferably, the device, in particular device body, preferably the first structure, second structure and/or the optional third structure, further comprises a coating or layer comprising an anti-proliferative agent, i.e. an anti-proliferative coating or layer.

Preferably, the anti-proliferative agent, in particular the coating or layer comprising an anti-proliferative agent, is arranged on an abluminal side of the second structure.

The term "abluminal side of the second structure" as used according to the present invention means the side of the second structure which - after implantation of the device, in particular device body, - is away from a bodily lumen, i.e. is directed to a wall containing a bodily lumen.

Alternatively, the anti-proliferative agent, in particular the coating or layer comprising an anti-proliferative agent, is arranged between the first structure and the second structure.

More specifically, the first structure and the second structure may be joined together by means of the anti-proliferative agent, in particular by means of the coating or layer comprising an anti-proliferative agent. In other words, it may be preferred that the anti-proliferative agent, in particular the coating or layer comprising an anti-proliferative agent, is arranged on an abluminal side of the first structure and is concurrently arranged on a luminal side of the second structure.

The anti-proliferative agent is in particular selected from the group comprising limus derivatives, sirolimus, everolimus, biolimus A9, tacrolimus, zotarolimus, paclitaxel, docetaxel, methotrexate, azathioprine, vincristine, vinblastine, fluorouracil, doxorubicinhydrochloride, mitomycin and mixtures thereof.

In a further embodiment, the coating or layer, which comprises an anti-proliferative agent, further comprises an excipient. In other words, according to a further embodiment, the device, in particular device body, preferably the first structure, second structure and/or the optional third structure, further comprises a coating or layer comprising an anti-proliferative agent and an excipient.

The excipient may be selected from the group comprising probucol, polyvinyl pyrrolidone, glycerine, polyhydroxyethyl, methacrylates, polyethylene glycole, polypropylene glycole, butylated hydroxytoluene (BHT), resveratol, polyvinyl alcohol, polydioxanone, polycaprolactone, polyglu-conate, poly(lactic acid)polyethylene oxide copolymer, modified cellulose, polyhydroxybutyrate, polyamino acids, polyphosphate esters, polyvalerolactones, poly-e-decalactones, polylactonic acid, polyglycolic acid polylactides, polyglycolides, copolymers of the polylactides and polygly-colides, poly-e caprolactone, polyhydroxybutyric acid, polyhydroxybutyrates, polyhy-droxyvalerates, polyhydroxybutyrate-co-valerates, poly(1,4-dioxane-2,3-dione), poly(1,3-dioxane-2-one), poly-para-dioxanones, polyanhydrides, polymaleic acid anhydrides, polyhy-droxy methacrylates, fibrin, polycyanoacrylates, polycaprolactone dimethylacrylates, poly-b-maleic acid polycaprolactone butyl acrylates, multiblock polymers from oligocaprolactonediols and oligodioxanonediols, polyether ester multiblock polymers from PEG and polybutylene terephthalate, polypivotolactones, polyglycolic acid trimethyl carbonates, polycaprolactone, glycolides, poly(g-ethyl glutamate), poly(DTH-iminocarbonate), poly(DTE-co-DT-carbonate), poly(bisphenol A-iminocarbonate), polyorthoesters, polyglycolic acid trimethyl carbonates, poly-trimethyl carbonates polyiminocarbonates, poly(N-vinyl)-pyrrolidone, polyvinyl alcohols, polyester amides, glycolized polyesters, polyphosphoesters, polyphosphazenes, poly[p-carboxyphenoxy)propane], polyhydroxy pentanoic acid, polyanhydrides, polyethylene oxide propylene oxide, soft polyurethanes, polyurethanes having amino acid residues in the backbone, polyetheresters such as polyethylene oxide, polyalkene oxalates, polyorthoesters as well as copolymers thereof, lipids, waxes, oils, polyunsaturated fatty acids, eicosapentaenoic acid, timnodonic acid, docosahexaenoic acid, arachidonic acid, linoleic acid, alpha-linolenic acid, gamma-linolenic acid, carrageenans, fibrinogen, agar-agar, starch, collagen, protein based polymers, polyamino acids, synthetic polyamino acids, zein, polyhydroxyalkanoates, pectic acid, actinic acid, carboxymethyl sulfate, albumin, hyaluronic acid, chitosan and its derivatives, heparan sulfates and its derivates, heparins, chondroitin sulfate, dextran, beta-cyclodextrins, copolymers with PEG and polypropylene glycol, gum arabic, guar, gelatin, collagen, collagen N-hydroxysuccinimide, lipids, phospholipids, polyacrylic acid, polyacrylates, polymethyl methacrylate, polybutyl methacrylate, polyacrylamide, polyacrylonitriles, polyamides, polyetheramides, polyethylene amine, polyimides, polycarbonates, polycarbourethanes, polyvinyl ketones, polyvinyl halogenides, polyvinylidene halogenides, polyvinyl ethers, polyisobutylenes, polyvinyl aromatics, polyvinyl esters, polyvinyl pyrrolidones, polyoxymethylenes, polytetramethylene oxide, polyethylene, polypropylene, polytetrafluoroethylene, polyurethanes, polyether urethanes, silicone polyether urethanes, silicone polyurethanes, silicone polycarbonate urethanes, polyolefin elastomers, polyisobutylenes, fluorosilicones, carboxymethyl chitosans, polyaryletheretherketones, polyetheretherketones, polyethylene terephthalate, polyvalerates, carboxymethylcellulose, cellulose, rayon, rayon triacetates, cellulose nitrates, cellulose acetates, hydroxyethyl cellulose, cellulose butyrates, cellulose acetate butyrates, ethyl vinyl acetate copolymers, polysulfones, epoxy resins, ABS resins, EPDM gums, silicones such as polysiloxanes, polydimethylsiloxanes, polyvinyl halogens, cellulose ethers, cellulose triacetates, shellac, poly-paraxylylenes, copolymers of the aforementioned polymers and mixtures thereof.

In a further embodiment, the device body may consist of the first structure, the second structure, the anti-proliferative agent, in particular the coating or layer comprising an anti-proliferative agent and an optional excipient, and optionally of additives. With respect to the first structure, the second structure, the anti-proliferative agent, in particular the coating or layer comprising an anti-proliferative agent and optionally an excipient, reference is made to the previous description. With respect to useful additives, reference is made to the additives as described in the following.

In a further embodiment, the device, in particular the device body, the first structure, the second structure, the optional third structure and/or the optional coating or layer comprising an anti-proliferative agent and optionally an excipient, comprises (further) additives which are preferably selected from the group comprising radio-opaque additives, antimicrobial, more particularly antibiotic, additives, wound healing-promoting additives, disinfecting additives, anti-inflammatory additives, growth factors, cell-differentiating factors, cell-adhesive factors, cell-recruiting factors, cell receptors, cell-binding factors, cytokines, peptides, structural proteins, extracellular proteins such as, for example, collagen, serum proteins such as, for example, albumin, polysaccharides such as, for example, hyaluronic acid, oligonucleotides, polynucleotides, DNA, RNA, salts thereof, stereoisomers, more particularly diastereomers, thereof and mixtures thereof.

In a further embodiment, the device body may consist of the first structure, the second structure, the optional third structure, the optional anti-proliferative agent, in particular the optional coating or layer comprising an anti-proliferative agent and optionally an excipient, and optionally of additives. With respect to the first structure, the second structure, the optional third structure, the optional anti-proliferative agent, in particular the optional coating or layer comprising an anti-proliferative agent and optionally an excipient, and the optional additives, reference is made to the above description.

According to a preferred embodiment, from a luminal side to an abluminal side, the device, in particular device body, comprises the following order of structures:
a) a first structure comprising at least one metal, preferably at least one bioerodible metal, selected from the group comprising magnesium, zinc, iron and alloys thereof,
b) a second structure comprising at least one polymer selected from the group comprising polylactide, polycaprolactone, poly(trimethylene carbonate), polyhydroxylalkanoates, polyglycolic acid, polyhydroxybutyrate, poly(3-polyhydroxybutyrate), poly(4-polyhydro-xybutyrate), copolymers thereof, and blends thereof,
c) a third structure comprising a connecting material and
d) a coating or layer comprising an anti-proliferative agent and optionally an excipient.

With respect to the first structure, the at least one metal, the second structure, the at least one polymer, the third structure, the coating or layer comprising an anti-proliferative agent and optionally an excipient, reference is made to the previous description.

In a further embodiment, from a luminal side to an abluminal side, the device, in particular the device body, comprises the following orders of layers:
a) a layer of a first structure comprising at least one metal, preferably at least one one bio-erodible metal, selected from the group comprising magnesium, zinc, iron and alloys thereof,
b) a layer of a second structure comprising at least one polymer, preferably bioerodible polymer, selected from the group comprising polylactide, polycaprolactone, poly(trimethylene carbonate), polyhydroxyalkanoates, polyglycolic acid, polyhydroxybutyrate, poly(3-hydroxybutyrate), poly(4- hydroxybutyrate), copolymers thereof, and blends thereof.

With respect to the layers/coating, in particular the first structure, the at least one metal, the second structure, the at least one polymer, the third structure, the anti-proliferative agent and the optional excipient, reference is made to the previous description.

According to an especially preferred embodiment, from a luminal side to an abluminal side, the device, in particular device body, comprises the following order of structures:
a) a first structure comprising magnesium and/or magnesium alloy,
b) a second structure comprising polylactide and/or a copolymer containing lactic acid units,
c) a third structure comprising a connecting material and
d) a coating or layer comprising an anti-proliferative agent and optionally an excipient.

With respect to the first structure, the second structure, the third structure, the layer comprising an anti-proliferative agent and optionally an excipient, reference is made to the previous description.

In particular, from a luminal side to an abluminal side, the device, in particular device body, comprises the following order of layers:
a) a layer of a first structure comprising magnesium and/or magnesium alloy,
b) a layer of a second structure comprising polylactide and/or a copolymer containing lactic acid units,
c) a layer of a third structure comprising a connecting material and
d) a coating or layer comprising an anti-proliferative agent and optionally an excipient.

With respect to the layers/coating, in particular the first structure, the second structure, the third structure, the anti-proliferative agent and the optional excipient, reference is made to the previous description.

In a further embodiment, the device, in particular device body, may have a cylindrical, in particular tubular, shape.

In an alternative embodiment the device, in particular device body, may have a spiral or helical, in particular helical, shape.

Preferably, the device body includes a pattern of a plurality of structural elements, in particular interconnecting structural elements. The pattern of structural elements can take on a variety of patterns. The variations in the structure of patterns are virtually unlimited.

Preferably, the pattern of a plurality of structural elements is constituted by the first structure, the second structure and at least one member selected from the group comprising a third structure, an anti-proliferative agent, in particular a coating or layer comprising an anti-proliferative agent and optionally an excipient, additives and combinations thereof. With respect to the first structure, the second structure, the third structure, the anti-proliferative agent, in particular coating or layer comprising an anti-proliferative agent and optionally an excipient, and additives, reference is made to the previous description.

Further, the device body may comprise or be arranged as a lattice structure, in particular textile lattice structure, which is composed of structural elements, in particular interconnecting structural elements.

Preferably, the lattice structure is constituted by the first structure, the second structure and at least one member selected from the group comprising a third structure, an anti-proliferative agent, in particular a coating or layer comprising an anti-proliferative agent and optionally an excipient, additives and combinations thereof. With respect to the first structure, the second structure, the third structure, the anti-proliferative agent, in particular coating or layer comprising an anti-proliferative agent and optionally an excipient, and additives, reference is made to the previous description.

The structural elements as mentioned in the preceding paragraphs are preferably selected from the group comprising struts, in particular linking struts, bending elements and combinations thereof.

In a further embodiment, the device body comprises or is a spiral (coil) or helix, preferably helix. In particular, the spiral or helix is sinusoidal in its circumferential direction. Preferably, the spiral or helix is constituted by the first structure, the second structure and at least one member selected from the group comprising a third structure, an anti-proliferative agent, in particular a coating or layer comprising an anti-proliferative agent and optionally an excipient, additives and combinations thereof. With respect to the first structure, the second structure, the third structure, the anti-proliferative agent, in particular coating or layer comprising an anti-proliferative agent and optionally an excipient, and additives, reference is made to the previous description.

According to a preferred embodiment, the device according to the present invention is an endovascular, in particular endovenous or endoarterial, more preferably endoarterial, device.

According to an especially preferred embodiment, the device according to the present invention is a stent, in particular an endovascular stent, in particular an endovenous or endoarterial stent, more preferably an endoarterial stent. The stent may in particular be a self-expandable stent or a balloon-expandable stent.

Accordingly, the device body is preferably a stent body, in particular an endovascular stent body, preferably an endovenous or endoarterial stent body, more preferably an endoarterial stent body. Further, the device body may be a self-expandable stent body or a balloonexpandable stent body.

According to a second aspect, the invention relates to a medical kit or system, in particular for use in angioplasty. The kit or system comprises a device, in particular an endoluminal device, according to the first aspect of the invention. Additionally, the kit or system comprises a delivery device.

In a preferred embodiment, the endoluminal device is attached to the delivery device, in particular crimped onto the delivery device.

The delivery device is preferably a catheter, in particular a balloon catheter.

With respect to further features and advantages of the kit and system, respectively, in particular the endoluminal device, reference is made to the embodiments described under the first aspect of the invention.

In the following, the present invention will be illustrated in more detail by a disclosure of preferred embodiments presented in examples. In the embodiments, individual features of the invention may be realized exclusively or in combination with other features. Any described embodiment is given for the sole purpose of illustration and better understanding of the invention, and is in no way to be interpreted as a limitation.

### MANUFACTURING EXAMPLES

### Example 1: Manufacture of a sirolimus coated stent

At first, a tube of magnesium was drawn. Then, poly(L-lactide) was extruded onto the drawn tube. After the extrusion, a pattern of a plurality of interconnecting structural elements was formed on the extruded tube by laser cutting using a femtosecond laser. However, it is clear that also alternative lasers such as excimer, carbon dioxide or YAG can be used for forming the pattern. After the laser cutting, the stent was crimped onto a balloon of a balloon catheter. Thereafter, the stent was coated with sirolimus and probucol.

### Example 2: Manufacture of a magnesium-poly(L-lactide) stent

A sheet of magnesium and a sheet of poly(L-lactide) were glued by means of poly(L-lactide-co-glycolide). Afterwards, the composite structure was subjected to chemical etching. After chemical etching, the composite structure was rolled and glued to form a stent structure. For gluing the rolled composite structure, poly(L-lactide-co-glycolide) was also applied.

### Example 3: Manufacture of a magnesium wire/poly(L-lactide) stent

A tube of poly(L-lactide) was cut by means of a femtosecond laser. Subsequently, the interior side of the tube was provided with a magnesium wire.

### FIGURES

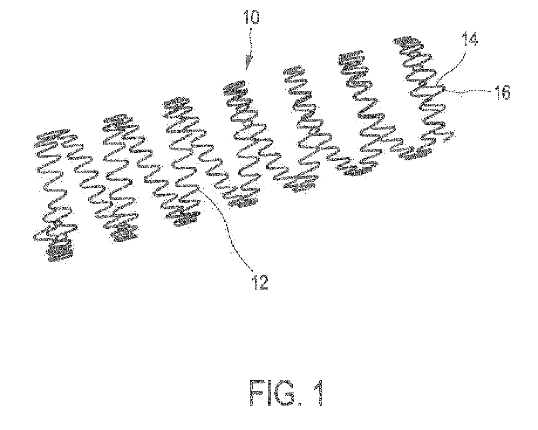

Fig. 1 discloses an endoluminal device 10 of the present invention.

The endoluminal device 10 has a device body 12 in the form of a helix which is sinusoidal in its circumferential direction. The helix 12 comprises struts 14 and bending elements 16.

The helix 12 can be constituted by one or more magnesium wires and by one or more yarns made of poly(L-lactide) such that the magnesium wire and magnesium wires, respectively, are arranged at an interior (luminal) side of the device 10, while the poly(L-lactide) yarn and poly(L-lactide) yarns, respectively, are arranged at an exterior (abluminal) side of the device 10.

The magnesium wire/wires and poly(L-lactide) yarn/yarns may be connected to each other by means of an additional yarn of poly(D-lactide), by an additional magnesium wire or by a combination thereof. Alternatively, the magnesium wire/wires and the poly(L-lactide) yarn/yarns can be glued, for example by means of poly(D,L-lactide).

The endoluminal device 10 is preferably a stent.

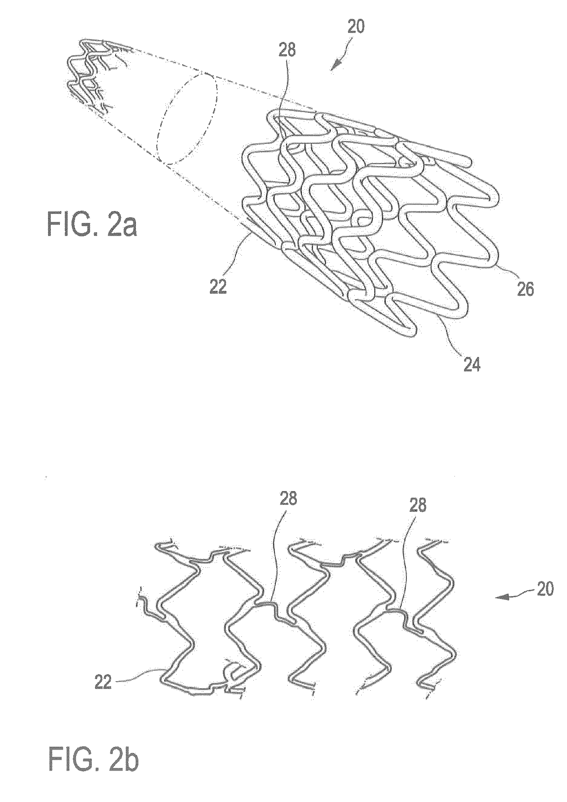

Fig. 2 shows a further endoluminal device 20 according to the present invention.

The endoluminal device 20 has a device body 22 which is a lattice structure. The lattice structure 22 is composed of a plurality of interconnecting structural elements. The structural elements comprise struts 24, bending elements 26 and linking struts 28.

The device body 22 may also be described as a lattice structure of successively arranged sinusoidal rings which are linked to each other by means of linking struts 28 (see also Fig. 2b).

The lattice structure 22 is constituted by a lattice structure of magnesium, a layer of poly(L-lactide-co-glycolic acid), a layer of poly(L-lactide) and an anti-proliferative coating, wherein poly(L-lactide-co-glycolic acid) layers the exterior surface of the magnesium lattice structure, polylactide layers the exterior surface of poly(L-lactide-co-glycolic acid) and the anti-proliferative coating layers the exterior surface of poly(L-lactide). The anti-proliferative coating may comprise sirolimus and probucol.

Thus, from a luminal to an abluminal side, the device 20 comprises the following order of structures:
a) a lattice of magnesium,
b) a layer of poly(L-lactide-co-glycolic acid),
c) a layer of poly(L-lactide) and
d) an anti-proliferative coating.

The device 20 is preferably a stent.

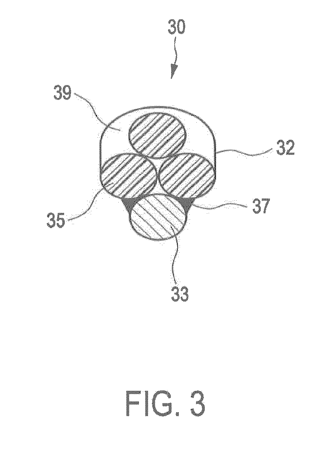

Fig. 3 discloses a cross-section of a further endoluminal device 30 having a device body 32. The device body 32 is constituted by a first structure 33, a second structure 35, a third structure 37 and an anti-proliferative coating 39.

The first structure 33 is arranged as a magnesium wire. The second structure 35 comprises three yarns made of poly(L-lactide).The third structure is made of poly(D,L-lactide) and is present in the form of a layer. Poly(D,L-lactide) acts as an adhesion promoting material which facilitates a connection between the first structure 33 and second structure 35. The anti-proliferative coating 39 comprises paclitaxel and preferably probucol. The anti-proliferative coating 39 layers the second structure 35.

The first structure 33 is arranged at a luminal side of the device 30. This means that the first structure 33 - after implantation of the device 30 - faces a bodily lumen, preferably an arterial lumen. The anti-proliferative coating 39 is arranged at an abluminal side of the device 30. This means that the coating 39 - after implantation of the device 30 - is away from the bodily lumen and is directed to the wall containing the lumen.

## Claims

1. Endoluminal device comprising a device body comprising a
- a first structure comprising at least one metal selected from the group comprising magnesium, zinc, iron and alloys thereof and
- a second structure comprising a least one polymer selected from the group comprising polylactide, polycaprolactone, poly(trimethylene carbonate), copolymers thereof, and blends thereof.

2. Endoluminal device according to claim 1, **characterized in that** the first structure and/or the second structure comprise at least one filamentary element, wherein the at least one filamentary element is preferably selected from the group comprising monofilament, staple fiber, pseudo monofilament, multifilament, thread, yarn, wire and combinations thereof.

3. Endoluminal device according to claim 1 or 2, **characterized in that** the first structure and/or the second structure comprise a textile structure, preferably a knitted fabric or braid.

4. Endoluminal device according to any of the preceding claims, **characterized in that** the first structure comprises magnesium and/or magnesium alloy.

5. Endoluminal device according to any of the preceding claims, **characterized in that** the second structure comprises polylactide, in particular selected from the group comprising poly(L-lactide), poly(D,L-lactide), poly(D-lactide) and mixtures thereof.

6. Endoluminal device according to any of the preceding claims, **characterized in that** the first structure is arranged at a luminal side of the device body.

7. Endoluminal device according to any of the preceding claims, **characterized in that** the second structure is arranged at an abluminal side of the device body.

8. Endoluminal device according to any of the claims 1 to 5, **characterized in that** the first structure and the second structure are alternately arranged on a luminal side and an abluminal side of the device body.

9. Endoluminal device according to any of the preceding claims, **characterized in that** the device further comprises a third structure comprising a connecting material, wherein the first and second structure are preferably joined together by means of the third structure.

10. Endoluminal device according to claim 9, **characterized in that** the connecting material is selected from the group comprising polyhydroxyalkanoates, poly(L-lactide), poly(D,L-lactide, poly(D-lactide), poly(L-lactide-co-glycolide), poly(D,L-lactide-co-glycolide), poly(D-lactide-co-glycolide), poly(L-lactide-co-caprolactone), poly(D-lactide-co-caprolactone), poly(D,L-lactide-co-caprolactone), polyglycolide, polyhydroxybutyrate, poly(3-hydroxybutyrate), poly(4-hydroxybutyrate), poly(3-hydroxybutyrate-co-3-hydroxyvalerate), poly(3-hydroxybutyrate-co-4-hydroxybutyrate), poly(3-hydroxybutyrate-co-3-hydroxyoctanoate) and mixtures thereof.

11. Endoluminal device according to any of the preceding claims, **characterized in that** the device further comprises an anti-proliferative agent, in particular a coating comprising an anti-proliferative agent and optionally an excipient.

12. Endoluminal device according to claim 11, **characterized in that** the coating is arranged at an abluminal side of the second structure.

13. Endoluminal device according to claim 11 or 12, **characterized in that** the anti-proliferative agent is selected from the group comprising limus derivatives, sirolimus, everolimus, biolimus A9, tacrolimus, zotarolimus, paclitaxel, docetaxel, methotrexate, azathioprine, vincristine, vinblastine, fluorouracil, doxorubicinhydrochloride, mitomycin and mixtures thereof.

14. Endoluminal device according to any of the preceding claims, **characterized in that** the device, from a luminal side to an abluminal side, has the following order of structures:
a) a first structure comprising magnesium and/or magnesium alloy,
b) a second structure comprising polylactide and/or a copolymer containing lactic acidunits,
c) a third structure comprising a connecting material, and
d) a coating comprising an anti-proliferative agent and optionally an excipient.

15. Endoluminal device according to any of the preceding claims, **characterized in that** the device is a stent, preferably an endovascular stent, in particular a self-expandable stent or a balloon-expandable stent.
